# EUROPÄISCHE PATENTSCHRIFT

(11) **EP 1 504 731 B1**
(45) Veröffentlichungstag und Bekanntmachung des Hinweises auf die Patenterteilung: **30.08.2006**
(21) Anmeldenummer: 04016386.7
(22) Anmeldetag: 13.07.2004
(51) Int. Cl.: A61F 2/20

(54) **Stimmprothese**
Voice prosthesis
Prothèse vocale

(30) Priorität: 16.07.2003 DE 20310919 U
(43) Veröffentlichungstag der Anmeldung: 09.02.2005
(73) Patentinhaber: ISKIA GmbH & Co. KG, 38829 Harsleben (DE)
(72) Erfinder: Neubauer, Norbert, 38820 Halberstadt (DE); Wienzek, Gerhard, 38820 Halberstadt (DE)
(74) Vertreter: Pfeiffer, Rolf-Gerd

(56) Entgegenhaltungen:
- US-A- 4 435 853
- US-A- 5 578 083
- US-A- 5 935 165

## Beschreibung

Die Erfindung betrifft eine Stimmprothese zum Einsatz in den Shunt zwischen Trachea und Oesophagus eines laryngektomierten Patienten, bestehend aus einem zylindrischen röhrenförmigen Grundkörper mit einer in der Luftröhre endenden Einlassöffnung und einer in der Speiseröhre endenden Auslassöffnung und mit einem im Bereich der Auslassöffnung innerhalb des Grundkörpers angeordneten ringförmigen Ventilsitz, dessen mittige kreisförmige Durchgangsöffnung durch eine in Form eines Einwegventils als Rückschlagventil wirkende Klappe geöffnet und verschlossen wird, einen am Grundkörper auslassseitig angeordneten seitlich abstehenden ersten äußeren Flansch zur Anlage an der Innenwandung der Speiseröhre und einen am Grundkörper einlassseitig angeordneten seitlich abstehenden zweiten äußeren Flansch zur Anlage an der Innenwandung der Luftröhre.

Bei einer Erkrankung mit operativer Entfernung des Kehlkopfs geht die Sprechfähigkeit verloren. Um im begrenzten Umfang die Sprechfähigkeit wieder herzustellen, wird in eine die Luftröhre mit der Speiseröhre verbindende chirurgische Fistel eine Stimmprothese eingesetzt. Diese Stimmprothese ist mit einem klappenartigen Rückschlagventil mit einer membranartigen Klappe oder Scheibe ausgebildet, die bei einem bestimmten ausreichend großen Öffnungsdruck in der Luftröhre einen Luftübertritt aus der Luftröhre in die Speiseröhre ermöglicht, in der Gegenrichtung jedoch keinen Transport des Speiseröhreninhalts (weder gasförmig, flüssig oder fest) zulässt. Der Öffnungswiderstand der Klappe oder Scheibe ist im Idealfall dabei so ausgewählt, dass der Öffnungsdruck dem Normaldruck eines Kehlkopfes entspricht.

Eine Stimmprothese dieser Art ist in der EP 0 093 567 B1 (US-A-4 435 853) und in der EP 0 132 957 B1 (US-A-4 610 691 bzw. AT- E 30 377 B als Übersetzung) offenbart. Diese Stimmprothese umfasst ein zylindrisches Gehäuse mit einer radialen Einlassöffnung und einer axialen Auslassöffnung und sie wird so eingesetzt, dass die Einlassöffnung in die Luftröhre und die Auslassöffnung in die Speiseröhre mündet. Die Auslassöffnung ist mit einem Einwegventil in Form einer senkrechten, innerhalb des Gehäuses gelenkig (schwenkbar) gelagerten Membran versehen. Durch das ganze Gehäuse hindurch erstreckt sich eine Bohrung, die an der Stelle, an welcher die Membran angelenkt ist, mit einem radial verlaufenden Rand oder Steg versehen ist, welcher als Schließ- oder Abdichtstruktur für die Membran wirkt. Der Innendurchmesser der Bohrung wird durch den Rand oder Steg erheblich verkleinert, woraus sich ein verringertes Strömungsvolumen der Prothese ergibt. Die gewählte Ventilart gewährleistet einen niedrigen Öffnungsdruck, doch ist die Montage der Prothese kompliziert und die Montageanforderungen sind sehr hoch, um zu verhindern, dass die Membran abfällt (sich löst), und auch um sicherzustelllen, dass die Membran gegen die Abdichtstruktur vollständig abdichtet. Das zylindrische Gehäuse dieser bekannten Stimmprothesen ist mit einer so ausreichenden axialen Länge ausgebildet, dass bei eingeschobener Stimmprothese diese mit mindestens einem radial sich von der Einlassseite der Stimmprothese aus erstreckenden Streifen außen am Hals des Trägers befestigt werden kann.

Eine zweite aus der EP 0 132 957 bekannte Ausführungsform einer Stimmprothesenvorrichtung, die für den Einsatz in die Fistel eines kehlkopfexstirpierten Patienten vorgesehen ist, ist mit einem axial kürzeren zylindrischen Gehäuse ausgebildet, da sie nur die chirurgische Fistel zwischen nebeneinander liegenden Wänden der Luftröhre und Speiseröhre mit kleinen Ansätzen an gegenüberliegen Enden zu überbrücken braucht. Das in der Speiseröhre liegende Ende des Grundgehäuses mit der axialen Auslassöffnung weist an seinem Au-ßenumfang einen flach vorspringenden Halteflansch auf, während an der Einlassseite des Grundgehäuses mit gleichfalls axial angeordneter Einlassöffnung statt der oben genannten Streifen zur Befestigung am Hals des Trägers ein kegelstumpfförmig ausgebildeter Halteflansch in Form einer Tellerfeder am Au-ßenumfang angeordnet ist, der innen an der Wand der Luftröhre anliegt. Die an der Auslassseite der Stimmprothesenvorrichtung angeordnete biegsame Ventilklappe ist zur Verbindung mit dem Grundgehäuse mit einer verlängernden Lasche in eine keilförmige Öffnung des Grundgehäuses gesteckt und dort mit Zement befestigt.

Eine andere Ausführungsform einer Stimmprothese ist von der Firma ENTER-MED; P.O. Box 236, 3440 Al Woerden, Niederlande, auf den Markt gebracht worden. Diese Prothese umfasst ein zylindrisches Gehäuse mit axialer Einlass-und Auslassöffnung. Das Einlassende ist mit einem ringförmigen äußeren Flansch zur Anlage an der Innenwand der Luftröhre versehen und das Auslassende weist ein Kugelventil zum Verschluss der Auslassöffnung auf. Die Kugel (ein Kunststoffball) des Kugelventils wird von axial beweglichen Klammern gehalten, die so dimensioniert sind, dass sie gleichzeitig eine Haltefunktion des Auslassendes an der Innenwand der Speiseröhre übernehmen. Bei dieser Art einer Stimmprothese ist ein sehr hoher Öffnungsdruck nötig; ferner ist das Ventil anfällig für Muskelbewegungen in der Speiseröhre, z. B. beim Schlucken.

Ferner ist aus der DE 690 03 817 T2 eine Stimmprothese bekannt, umfassend ein zylindrisches Mittelstück, einen von einem Ende des Mittelstücks abstehenden ersten äußeren Flansch, einen von einem zweiten Ende des Mittelstücks abstehenden zweiten äußeren Flansch und einen im ersten Ende des Mittelstücks angeordneten Ventilsitz mit einem zylindrischen Durchgang durch das Mittelstück, der von einer Ventilklappe geöffnet und verschlossen wird. Das Mittelstück und die Flansche sind aus einem flexiblen Werkstoff (Silikon) geformt, wobei auch die Ventilklappe mit dem Mittelstück materialeinheitlich ausgebildet ist. Zur Versteifung des flexiblen Mittelstücks ist innerhalb des Mittelstücks in der Nähe des Ventilsitzes ein Ring angeordnet, der im Verhältnis zum flexiblen Material des Mittelstücks steif ist. Die Einbettung dieses Versteifungsrings birgt die Gefahr in sich, dass dieser Ring sich aus dem Silikon mit Risiko für den Patienten lösen kann. Der starre obere Flansch erfordert beim Einlegen der Stimmprothese in die Fistel eine komplizierte Technik, bei der unter Einsatz einer Spreizhülse und praktisch ohne Sichtkontrolle die Stimmprothese platziert werden muss, wobei die Gefahr der Fistelaufweitung besteht. Die Ventilklappe dichtet auf einer Fläche ab. Hier besteht das Risiko einer Verkrustung und Störung der absoluten Abdichtung. Der schmale Versteifungsring birgt eine Verrutschungsgefahr, da er nicht mit den Flanschen stoffschlüssig verbunden ist. Durch den schmalen Versteifungsring bewirken Muskelbewegungen und Muskeldrücke negative Auswirkungen auf die Ventilfunktion.

Bei den beschriebenen bekannten Stimmprothesen sind jeweils der Grundkörper und die Halteflansche aus dem gleichen Material gefertigt. Da für die Flansche eine gewisse Flexibilität gefordert wird, bedingt dies auch einen flexiblen Grundkörper mit insbesondere dem dann bestehenden Nachteil, dass der Grundkörper auf Grund seiner geringen Härte für mechanische Beschädigungen, beispielsweise beim Reinigen mit einer Bürste, anfälliger ist und durch die mechanischen Beschädigungen die Ansiedlung bzw. Anlagerung von Bakterien leichter möglich wird.

Ausgehend von diesem geschilderten Stand der Technik ist es Aufgabe der Erfindung, die Nachteile und Mängel der bisher bekannten Stimmprothesen weitgehend zu überwinden und unter Aufrechterhaltung eines niedrigen Öffnungsdrucks eine sichere Einweg-Ventilfunktion zu sichern. Zudem soll die Stimmprothese einfach herzustellen und ohne Gefahr einer unkontrollierten Fistelaufweitung einsetzbar sein.

Die gestellte Aufgabe wird mit den kennzeichnenden Merkmalen des Anspruchs 1 der Erfindung dadurch gelöst, dass der Grundkörper aus einem harten und starren Material und die Flansche sowie die Ventilklappe aus weichen elastischen Materialien bestehen. Die Verbindung der einzelnen Elemente kann in beliebiger Weise, beispielsweise durch Verklebung erfolgen. Besonders zweckmäßig werden jedoch der Grundkörper und die Flansche, vorzugsweise auch die Ventilklappe in einer Umspritzeinheit hergestellt.

Vorteilhafte Ausgestaltungen der Erfindung sind in den Unteransprüchen angegeben.

Durch das Merkmal der Erfindung, den Grundkörper aus einem starren harten Werkstoff herzustellen, wird es möglich, den Grundkörper mit variabler Wandstärke auszubilden und bei Einhaltung eines erforderlichen minimalen Innendurchmessers des Strömungskanals den Außendurchmesser des Grundkörpers gegenüber sonst üblichen Stimmprothesen für kleine Fistelgrößen zu verkleinern. Die Stimmprothese kann leichter und unter Sichtkontrolle platziert werden, der erforderliche Eingriff wird nicht so aufwändig und sowohl die Fistel als auch das Stoma können nun auch im Durchmesser kleiner ausgebildet sein. Falls erforderlich kann entgegengesetzt unter Beibehaltung des Außendurchmessers des Grundkörpers der Innendurchmesser und damit der Strömungskanal der Stimmprothese vergrößert werden. Weiterhin wird durch den kompakten, harten Grundkörper die Formstabilität der Stimmprothese gegen den in der Fistel herrschenden Verformungsdruck erhöht, wodurch ein Platzierungs- und Dichtungsvorteil gegeben ist. Zusätzlich ist durch die Verwendung eines harten Werkstoffs die Gefahr einer mechanischen Beschädigung der Innenfläche des Grundkörpers, beispielsweise bei der Reinigung mit einer Bürste, deutlich verringert.

Die Anwendung der Umspritztechnologie ermöglicht die erfindungsgemäße gleichzeitige Verwendung unterschiedlicher Materialien und damit die Herstellung einer einstückigen Stimmprothese aus unterschiedlichen Grundwerkstoffen. Der Grundkörper der Stimmprothese wird vorteilhaft dabei aus Polyethylen (PE) oder aus Polypropylen (PP) hergestellt, während die Flansche sowie die Ventilklappe aus einem thermoplastischen Elastomer (TPE) bestehen. Grundsätzlich können auch andere Materialkombinationen, z. B. Polyurethan hart (PUR - h) für den Grundkörper und Polyurethan weich (PUR - w) für die Flansche und Ventilklappe zum Einsatz kommen, wenn die betreffenden Materialkombinationen miteinander eine ausreichend feste Verbindung beim Umspritzen eingehen. Für die Flansche können jeweils auch unterschiedliche flexible Materialien aus der Gruppe der elastischen Thermoplaste verwendet werden. Ferner kann es sinnvoll sein, dass die Flansche und die Ventilklappe aus unterschiedlichen flexiblen Materialien bestehen.

Die Fertigung mit Hilfe der Umspritztechnologie geschieht dabei derart, dass die Flansche aus dem ausgewählten Material am starren Grundkörper angespritzt werden. Hierbei werden die Flansche erfindungsgemäß so angeordnet und ausgebildet, dass der Grundkörper auslassseitig durch einen axialen Teil des seitlich abstehenden ersten äußeren Flansches verlängert und einlassseitig durch den seitlich abstehenden zweiten äußeren Flansch unten abgeschlossen ist.

Die aus einem flexiblen Material bestehende Ventilklappe ist nach einer vorteilhaften Ausgestaltung der Erfindung aus einem einheitlichen Material gemeinsam mit dem ösophagus-seitigen Flansch mit Hilfe der Umspritztechnologie am starren Grundkörper befestigt. Nach einer weiteren Variante ist die mit einem zungenförmigen Ende ausgebildete Ventilklappe mit diesem Ende in einen Schlitz des Grundkörpers durch Einspritzen mit dem Grundkörper fest verbunden. Alternativ ist es aber auch möglich, dass das zungenförmige Ende der Ventilklappe durch den Schlitz des starren Grundkörpers gesteckt und hier mechanisch durch Kleben oder durch eine nietkopfähnliche thermische Verformung befestigt ist.

Der innerhalb des Grundkörpers im Bereich der Auslassöffnung angeordnete ringförmige Ventilsitz ist nach einer weiteren Ausgestaltung der Erfindung einteilig mit dem Grundkörper hergestellt, so dass dieser Ventilsitz gleichfalls aus dem harten und starren Material des Grundkörpers besteht. Zur Verbesserung der Dichtung der Ventilklappe können auf dem Ventilsitz Dichtungsrillen eingespritzt werden.

Weitere Einzelheiten, Vorteile und Merkmale der Erfindung werden nachfolgend an in schematischen Zeichnungsfiguren dargestellten Ausführungsbeispielen näher erläutert. Für gleiche Konstruktionsteile werden hierbei zur besseren Übersicht gleiche Bezugszeichen verwendet.

Es zeigen:
- Fig. 1: einen Vertikalschnitt durch eine erfindungsgemäße Stimmprothese,
- Fig. 2: einen vergrößerten Teilausschnitt der Ventilklappenbefestigung,
- Fig. 3: einen vergrößerten Teilausschnitt der Ventilklappenbefestigung in einer zweiten Ausbildungsvariante,
- Fig. 4: einen Vertikalschnitt durch eine zweite erfindungsgemäße Stimmprothese,
- Fig. 5: einen Vertikalschnitt durch eine dritte erfindungsgemäße Stimmprothese,
- Fig. 6: eine Ansicht der Fig. 4 und 5 von unten mit seitlich angeordneter Einführhilfe,
- Fig. 7: einen Einführungsstab.

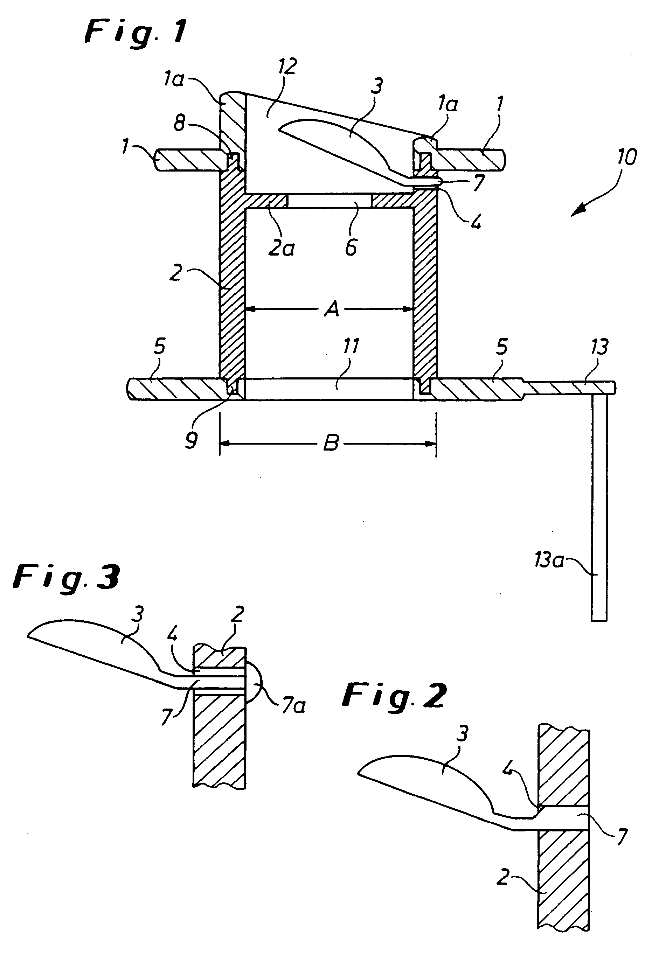

In Figur 1 ist eine komplette erfindungsgemäße Stimmprothese 10 in einem Vertikalschnitt dargestellt. Die Stimmprothese 10 besteht aus einem röhrenförmigen zylindrischen Grundkörper 2 aus einem harten starren Material mit einer Einlassöffnung 11 und einer Auslassöffnung 12. Im Bereich der Auslassöffnung 12 ist innen im Grundkörper 2 ein einstückig mit diesem gefertigter Ventilsitz 2a mit einer kreisförmigen Durchgangsöffnung 6 angeordnet. Die Durchgangsöffnung 6 wird durch eine auf dem Ventilsitz 2a aufliegende Ventilklappe 3 in der Schließstellung geschlossen (die Schließstellung ist nicht dargestellt) und durch Abheben vom Ventilsitz 2a wie dargestellt geöffnet. Damit die aus einem flexiblen Material gefertigte Ventilklappe 3 in Form eines Rückschlagventils wirken kann, ist sie mit einem zungenförmigen Ende 7 ausgebildet und mit diesem zungenförmigen Ende 7 in einen Schlitz 4 des Grundkörpers 2 gesteckt und dort befestigt bzw. eingespritzt.

An der Auslassseite des Grundkörpers 2 - in der Zeichnung oben - ist ein seitlich abstehender erster äußerer Flansch 1 angeordnet, der zur Anlage an der Innenwand der Speiseröhre (die Speiseröhre ist nicht dargestellt) dient. Der Flansch 1 ist mit einem axialen Teil 1 a ausgebildet, der den Grundkörper 2 auslassseitig (nach oben hin) asymmetrisch verlängert, in die Speiseröhre hineinragt und den Ventilsitz 2a und die Ventilklappe 3 beispielsweise vor Ablagerungen von Speiseresten schützt. Der aus einem flexiblen Material gefertigte Flansch 1 ist vorzugsweise durch Anwendung der Umspritztechnologie an dem oberen Rand 8 des Grundkörpers 2 angespritzt und dadurch praktisch nahtlos befestigt. Um eine optimale Befestigung zu gewährleisten, ist der Rand 8 als verjüngter Ringwulst ausgebildet, der in eine Ringnut im Flansch 1 eingepasst ist, wodurch eine zusätzliche formschlüssige Verbindung hergestellt wird.

An der Einlassseite des Grundkörpers 2 - in der Zeichnung unten - ist ein seitlich abstehender zweiter äußerer Flansch 5 angeordnet, der zur Anlage an der Innenwand der Luftröhre (die Luftröhre ist nicht dargestellt) dient. Dieser gleichfalls aus einem flexiblen Material gefertigte Flansch 5 schließt den unteren Rand 9 des Grundkörpers 2 nach außen ab (in der Zeichnung nach unten) und ist in gleicher Weise wie der Flansch 1 durch Anwendung der Umspritztechnologie an den Rand 9 des Grundkörpers 2 angespritzt. Auch hier ist durch eine Verzahnung des Randes 9 die Haftung des Flansches 5 am Grundkörper 2 optimiert.

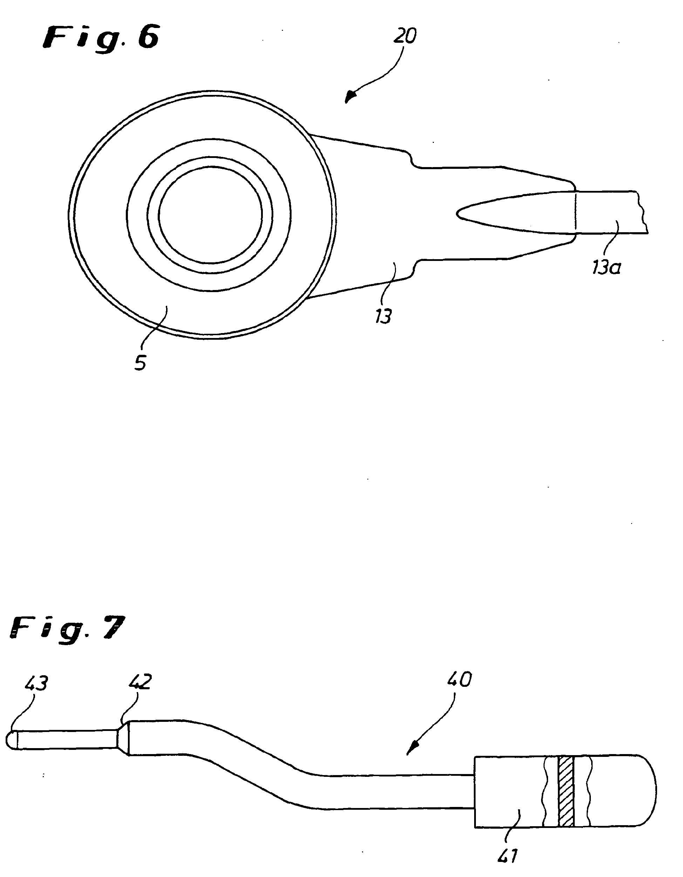

Zum Einsetzen der Stimmprothese 10 in die Fistel wird der Einführungsstab 40 (siehe Fig. 7) mit einem verkürzten Kopfende 43 verwendet. Zur Fixierung der Stimmprothese 10 auf dem Einführungsstab 40 besitzt der Flansch 5 an der Seite der Flanschverlängerung 13 einen axial nach außen (in der Zeichnung nach unten) gerichteten angespitzten Sicherungsfaden 13a, der nach dem Einsetzen der Stimmprothese 10 von der Flanschverlängerung 13 abgetrennt und mit dem Einführungsstab 40 nach dem Eingriff gemeinsam entfernt wird.

In den Figuren 2 und 3 ist in vergrößerten Abbildungen der Befestigungsbereich der Ventilklappe 3 im Schlitz 4 des Grundkörpers 2 dargestellt. In Fig. 2 ist das zungenförmige Ende 7 der Ventilklappe 3 in den Schlitz 4 des Grundkörpers 2 durch Anwendung der Umspritztechnologie eingespritzt und somit nahtlos mit dem Grundkörper 2 verbunden. In Fig. 3 ist alternativ zur Umspritztechnologie das zungenförmige Ende 7 der Ventilklappe 3 durch den Schlitz 4 des Grundkörpers 2 durchgesteckt und das überstehende Ende an der Außenseite des Grundkörpers 2 thermisch zu einem Nietkopf 7a verformt. Auch hierdurch ist eine feste Verbindung der Ventilklappe 3 mit dem Grundkörper gegeben.

Die in den Figuren 1 bis 3 dargestellte, aus unterschiedlichen Materialien bestehende, Stimmprothese zeichnet sich, insbesondere durch die Anwendung der Umspritztechnologie, durch eine einheitliche Bauform ohne vorstehende Kanten oder Nähte aus. Die erfindungsgemäße Verwendung eines harten starren Grundkörpers bei ansonsten weichen flexiblen Materialien der übrigen Bauteile ermöglicht dabei mit Vorteil eine deutliche Reduzierung des in Fig. 1 mit B bezeichneten Außendurchmessers bei gleichbleibendem Innendurchmesser A gegenüber den sonst üblichen Abmessungen bekannter Stimmprothesen.

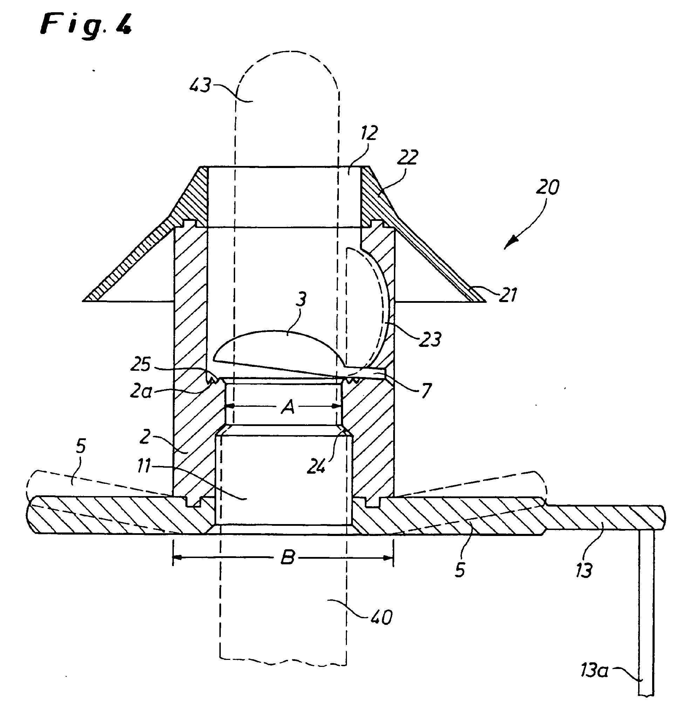

In Figur 4 ist eine komplette weitere erfindungsgemäße Stimmprothese 20 dargestellt. Diese Stimmprothese 20 besteht wie bei der Stimmprothese 10 der Fig. 1 aus einem röhrenförmigen zylindrischen Grundkörper 2 aus einem harten starren Material mit einer Einlassöffnung 11 und einer Auslassöffnung 12. Einlassseitig - in der Zeichnung unten - schließt auch hier ein seitlich abstehender äußerer Flansch 5 aus einem flexiblen Material, der zur Anlage an der Innenwand der Luftröhre dient, in gleicher Weise den Grundkörper 2 ab. In der Fig. 4 ist der äußere Flansch 5 rechtwinklig zum Grundkörper 2 ausgerichtet. Erfindungsgemäß kann dieser Flansch 5 zur besseren Anlage an der Innenwand der Luftröhre auch etwas nach oben abgewinkelt angeordnet sein, wie in Fig. 4 gestrichelt dargestellt ist. Auslassseitig - in der Zeichnung oben - ist als oberer Abschluss des Grundkörpers 2 ein kegeliger Schutzring 22 als Ventilklappenschutz angeordnet, der auch bei Verdrehung des Ventils einen ausreichenden Schutz garantiert. Der Schutzring 22 ist mit einem seitlich abstehenden äußeren Flansch 21 aus einem flexiblen Material ausgebildet, der zur Anlage an der Innenwand der Speiseröhre dient, jedoch nicht wie bei Fig. 1 radial abgewinkelt, sondern in diesem Ausführungsbeispiel spitzwinklig nach außen vorstehend ausgeführt ist. Diese schräge Ausführungskonstruktion ermöglicht eine gute Platzierung, wobei die Gefahr einer Überdehnung der Fistel beim Plazieren vermieden wird. Im Dichtungsbereich der Ventilklappe 3 ist der Ventilsitz 2a mit eingearbeiteten Dichtungszackenrillen 25 ausgebildet, wodurch gegenüber der entsprechenden glatten Fläche der Fig. 1 eine bessere Abdichtung gegen Speise- und Flüssigkeitsteilchen erreicht wird.

Zum Einsetzen der Stimmprothese 20 in die zuvor eingebrachte Trachea-Ösophagus-Fistel wird die Stimmprothese mit der trachea-seitigen Öffnung 11 des Grundkörpers 2 auf den Einführungsstab 40 aufgesetzt und mit dem an der Flanschverlängerung 13 angespitzten Sicherungsfaden 13a auf dem Einführungsstab 40 befestigt. Ein ringförmiger konischer Anschlag 42 (siehe Fig. 7) begrenzt beim Einschieben des Einführungsstabes 40 durch ein entsprechend konisch ausgebildetes Gegenlager 24 der Stimmprothese 20 den weiteren Einschiebeweg, so dass ein Durchstoßen des Einführungsstabes 40 vermieden wird. Das in dieser Stellung an der Auslassöffnung 12 herausragende Kopfende 43 des Einführungsstabes 40 könnte hierbei gleichzeitig als "Einführungsbougie" (Dehnsonde) dienen. Damit beim Einschieben des Einführungsstabes 40 die Ventilklappe 3 ausreichend ausweichen kann, ist innen der feste Grundkörper 2 mit einer der Form der Ventilklappe 3 entsprechenden Schutzkammer 23 ausgebildet. In der Fig. 4 sind der eingeschobene Einführungsstab 40 und die in die Schutzkammer 23 hochgeklappte Ventilklappe 3 gestrichelt eingezeichnet. Damit die Ventilklappe 3 auch in dieser Weise dem Einführungsstab 40 ausweichen kann, ist oberhalb des Ventilklappenendes 7 die Wanddicke des Grundkörpers 2 entsprechend reduziert.

Gegenüber der Stimmprothese 10 der Fig. 1, bei der die Ventilklappe 3 im oberen Bereich des Grundkörpers 2 angeordnet ist, ist bei der Stimmprothese 20 zum weiteren Schutz vor Speiseresten die Ventilklappe 3 tiefer in den Grundkörper 2 verlegt, wobei sich die Ventilklappe 3 in etwa in der Mitte des Grundkörpers 2 befindet.

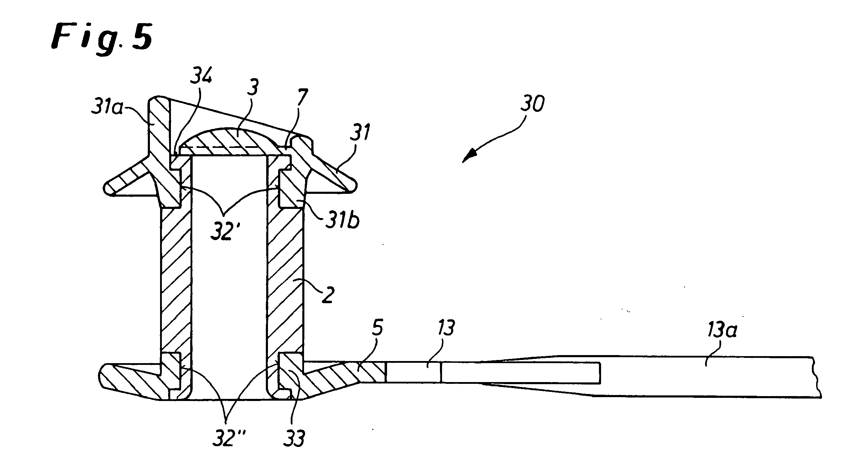

In Figur 5 ist die Variante einer weiteren kompletten Stimmprothese 30 der Erfindung in einem Vertikalschnitt dargestellt. In dieser Variante wird die Ventilklappe 3 material-einheitlich beim Spritzen des auslassseitigen Flansches 31 mit gefertigt und dabei direkt von der Stirnfläche 34 des Grundkörpers 2 abgeformt, wodurch ein optimales späteres Abdichtungsverhalten erzielt wird. Bei der Fertigung ist dabei zu beachten, dass vor dem Anspritzen des auslassseitigen Flansches 31 einschließlich der Ventilklappe 3 die Stirnfläche 34 des Grundkörpers 2 mit einem Trennmittel versehen werden muss, um eine unerwünschte feste Verbindung der Ventilklappe 3 mit dem Grundkörper 2 zu vermeiden.

In der Schließstellung liegt die Ventilklappe 3 auf dieser oberen Stirnfläche des Grundkörpers 2 auf, so dass damit der Ventilsitz 2a der Stimmprothesen 10 und 20 der Figuren 1 und 4 nicht mehr erforderlich ist. Die Stimmprothese 30 besitzt somit einen stufenfreien Innenraum, wodurch das laminare Strömungsverhalten in der Stimmprothese 30 günstiger wird und gleichzeitig die Voraussetzungen für ein noch besseres Verhältnis von Innen- zum Außendurchmesser des Grundkörpers geschaffen wird. Ein weiterer Vorteil ist, dass sich ein stufenloser Innenraum leichter reinigen lässt.

Die Befestigung der Flansche 5 und 31 am Grundkörper 2 geschieht nicht wie bei den Stimmprothesen 10 und 20 der Figuren 1 und 4 durch eine axiale Verzahnung, sondern durch eine ringförmige Einschnürung 31 b, 33 der Flansche, die in jeweils eine ringförmige Nut 32', 32" am Außenumfang des Grundkörpers 2 eingreifen. Durch eine derartige hinterschnitt-artige Gestaltung der Umspritzzonen am Grundkörper 2 werden die Festigkeiten der Verbindungen zwischen dem Grundkörper 2 und den Flanschbereichen deutlich verbessert.

In ähnlicher Weise wie bei der Stimmprothese 20 sind die Flansche 5, 31 der Stimmprothese 30 kegelstumpfartig nach innen gebogen, wobei durch die dünne Ausführung des ösophaguseigenen Flansches 31 auch hier ein vergleichsweise schonendes Einsetzen der Stimmprothese 30 in die Fistel möglich wird, ohne dass das Risiko einer ungewollten Dislokation ansteigt. Weiterhin gleicht diese Konstruktion kleinere Wanddickenunterschiede im Bereich der Fistel leichter aus als ein dickerer Flansch. Auch die Stimmprothese 30 ist mit einer Flanschverlängerung 13 versehen und kann mit Hilfe des Einführungsstabes 40 mit dann verkürztem Kopfende 43 in die Fistel eingebracht werden.

In der Figur 6 ist in einer Ansicht von unten für die Stimmprothesen 10, 20 und 30 die Gestaltung des Flansches 5 mit der Flanschverlängerung 13 und dem Sicherungsfaden 13a bei radialer Anordnung dargestellt.

Gemäß Figur 7 ist der Einführungsstab 40 zwischen dem ringförmigen konischen Anschlag 42 und einer Griffplatte 41 gekrümmt ausgebildet, wodurch beim Einsetzen der Stimmprothesen mit Hilfe des Einführungsstabes 40 eine bessere Sichtkontrolle gegeben ist. Der Einführungsstab 40 besitzt eine, in der Zeichnung nicht weiter dargestellte Öffnung, durch die der Sicherungsfaden 13a der Stimmprothesen vor dem Einsetzen gezogen wird, um die Stimmprothesen mit dem Einführungsstab 40 sicher zu verbinden.

Die Erfindung ist nicht auf die dargestellten Anwendungsbeispiele beschränkt, sondern bei allen bekannten Bauformen im Handel erhältlicher Stimmprothesen anwendbar, sofern das wesentliche Merkmal der Erfindung erhalten bleibt, die Stimmprothesen aus unterschiedlichen Materialien und den Grundkörper aus einem harten und starren Material zu fertigen.

### Bezugszeichenliste

- 1, 21, 31: oberer Flansch
- 1 a: axialer Teil von 1
- 2: Grundkörper
- 2a: Ventilsitz
- 3: Ventilklappe
- 4: Schlitz in 2
- 5: unterer Flansch
- 6: kreisförmige Öffnung in 2a
- 7: Ventilklappenende
- 7a: Nietkopf an 7
- 8: Verzahnung an 1
- 9: Verzahnung an 5
- 10, 20, 30: Stimmprothese
- 11: Einlassöffnung
- 12: Auslassöffnung
- 13: Flanschverlängerung
- 13a: Sicherungsfaden
- 22: kegeliger Schutzring
- 23: Schutzkammer für 3
- 24: konisches Gegenlager für 42
- 25: Dichtungszackenrillen
- 31 a: axialer Teil von 31
- 31 b: ringförmige Einschnürungen von 31
- 32', 32": ringförmige Nut in 2
- 33: ringförmige Einschnürung von 5
- 34: obere Stirnfläche von 2
- 40: Einführungsstab
- 41: Griffplatte
- 42: ringförmiger konischer Anschlag
- 43: Kopfende von 40
- A: Innendurchmesser von 2
- B: Außendurchmesser von 2

## Patentansprüche

1. Stimmprothese (10, 20, 30) zum Einsatz in den Shunt zwischen Trachea und Oesophagus eines laryngektomierten Patienten, bestehend aus einem zylindrischen röhrenförmigen Grundkörper (2) mit einer in der Luftröhre endenden Einlassöffnung (11) und einer in der Speiseröhre endenden Auslassöffnung (12) und mit einem im Bereich der Auslassöffnung (12) innerhalb des Grundkörpers (2) angeordneten ringförmigen Ventilsitz (2a), dessen mittige kreisförmige Durchgangsöffnung (6) durch eine als Rückschlagventil in Form eines Einwegventils wirkende Ventilklappe (3) geöffnet und verschlossen wird, einen am Grundkörper (2) auslassseitig angeordneten seitlich abstehenden ersten äußeren Flansch (1) zur Anlage an der Innenwandung der Speiseröhre und einen am Grundkörper (2) einlassseitig angeordneten seitlich abstehenden zweiten äußeren Flansch (5) zur Anlage an der Innenwandung der Luftröhre, **dadurch gekennzeichnet, dass** der Grundkörper (2) aus einem harten und starren Material und die Flansche (1, 5, 21, 31) sowie die Ventilklappe (3) aus weichen elastischen Materialien bestehen.

2. Stimmprothese (10, 20, 30) nach Anspruch 1, **dadurch gekennzeichnet, dass** der Grundkörper (2) und die Flansche (1, 5, 21, 31) in einer Umspritzeinheit hergestellt sind.

3. Stimmprothese (10, 20, 30) nach Anspruch 1 oder 2, **dadurch gekennzeichnet, dass** der Grundkörper (2) aus Polyurethan hart und die Flansche (1, 5, 21, 31) sowie die Ventilklappe (3) aus Polyurethan weich bestehen, wobei die Forderung, dass diese Materialkombination eine ausreichend feste Verbindung beim Umspritzen miteinander eingehen, erfüllt sein muss.

4. Stimmprothese (10, 20, 30) nach Anspruch 1 oder 2, **dadurch gekennzeichnet, dass** der Grundkörper (2) aus Polyethylen oder aus Polypropylen und die Flansche (1, 5; 21, 31) sowie die Ventilklappe (3) aus einem thermoplastischen Elastomer bestehen.

5. Stimmprothese (10, 20, 30) nach einem oder mehreren der Ansprüche 1 bis 4, **dadurch gekennzeichnet, dass** der erste Flansch (1, 21, 31) und der zweite Flansch (5) jeweils aus unterschiedlichen flexiblen Materialien bestehen.

6. Stimmprothese (10, 20, 30) nach einem oder mehreren der Ansprüche 1 bis 5, **dadurch gekennzeichnet, dass** die Flansche (1, 5, 21, 31) am starren Grundkörper (2) angespritzt sind.

7. Stimmprothese (10, 20, 30) nach einem oder mehreren der Ansprüche 1 bis 6, **dadurch gekennzeichnet, dass** der Grundkörper (2) auslassseitig durch einen axialen Teil (1a, 31a) des seitlich abstehenden ersten äußeren Flansches (1, 31) verlängert und einlassseitig durch den seitlich abstehenden zweiten äußeren Flansch (5) abgeschlossen wird.

8. Stimmprothese (10, 20, 30) nach einem oder mehreren der Ansprüche 1 bis 7, **dadurch gekennzeichnet, dass** der starre Grundkörper (2) mit variabler Wandstärke ausbildbar ist, um für kleine Fistelgrößen bei Einhaltung eines erforderlichen minimalen Innendurchmessers (A) den Grundkörper (2) mit einem möglichst kleinen Außendurchmesser (B) auszubilden.

9. Stimmprothese (10, 20) nach einem oder mehreren der Ansprüche 1 bis 8, **dadurch gekennzeichnet, dass** die Flansche (1, 5, 21) sowie die Ventilklappe (3) jeweils aus unterschiedlichen flexiblen Materialien hergestellt sind.

10. Stimmprothese (10, 20) nach einem oder mehreren der Ansprüche 1 bis 9, **dadurch gekennzeichnet, dass** die Ventilklappe (3) mit einem zungenförmigen Ende (7) ausgebildet ist, welches durch einen Schlitz (4) des starren Grundkörpers (2) gesteckt und hier mechanisch durch Kleben oder durch eine nietkopfähnliche thermische Verformung (7a) befestigt ist.

11. Stimmprothese (10, 20) nach einem oder mehreren der Ansprüche 1 bis 9, **dadurch gekennzeichnet, dass** die mit einem zungenförmigen Ende (7) ausgebildete Ventilklappe (3) durch Einspritzen in einen Schlitz (4) des starren Grundkörpers (2) befestigt ist.

12. Stimmprothese (10, 20) nach einem oder mehreren der Ansprüche 1 bis 11, **dadurch gekennzeichnet, dass** der ringförmige Ventilsitz (2a) einteilig mit dem Grundkörper (2) gefertigt ist.

13. Stimmprothese (20) nach einem oder mehreren der Ansprüche 1 bis 12, **dadurch gekennzeichnet, dass** der Grundkörper (2) auslassseitig durch einen kegelig ausgebildeten Schutzring (22) mit einem regenschirmartig spitzwinklig nach außen vorstehenden Flansch (21) aus einem besonders flexiblem Material begrenzt wird, der in jeder Lage der Stimmprothese im Shunt die Ventilklappe (3) und den Ventilssitz von äußeren Einflüssen schützt.

14. Stimmprothese (20) nach einem oder mehreren der Ansprüche 1 bis 13, **dadurch gekennzeichnet, dass** der Grundkörper (2) in seiner Innenwand im Bereich der Ventilklappe (3) oberhalb des Befestigungsschlitzes (4) eine Schutzkammer (23) zur Aufnahme der Ventilklappe (3) aufweist.

15. Stimmprothese (20) nach einem oder mehreren der Ansprüche 1 bis 14, **dadurch gekennzeichnet, dass** der Grundkörper (2) innen mit einem ringförmigen Gegenlager (24) zur Einschiebbegrenzung eines vorzugsweise gekrümmten Einführungsstabes (40) ausgebildet ist.

16. Stimmprothese (20) nach einem oder mehreren der Ansprüche 1 bis 15, **dadurch gekennzeichnet, dass** im Dichtungsbereich der Ventilklappe (3) der Ventilsitz (2a) mit eingearbeiteten Dichtungszackenrillen (25) ausgebildet ist.

17. Stimmprothese (30) nach einem oder mehreren der Ansprüche 1 bis 8, **dadurch gekennzeichnet, dass** die Ventilklappe (3) mit ihrem Ventilklappenende (7) material-einheitlich mit dem oberen auslassseitigen Flansch (31) durch Spritzen gefertigt und in der Schließstellung der Stimmprothese (30) auf der oberen Stirnfläche (34) des Grundkörpers (2) dichtend aufliegt.

18. Stimmprothese (30) nach Anspruch 17, **dadurch gekennzeichnet, dass** der Grundkörper (2) einen stufenfreien Innenraum aufweist.

19. Stimmprothese (30) nach Anspruch 17 oder 18, **dadurch gekennzeichnet, dass** der Grundkörper (2) zur Aufnahme einer ringförmigen Einschnürung (31 b, 33) der Flansche (5, 31) an seinem Außenumfang jeweils eine ringförmige Nut (32', 32") aufweist.

## Claims

1. Voice prosthesis (10, 20, 30) for insertion into the shunt between the trachea and the esophagus of a laryngectomee comprising a cylindrical tubular base body (2) with an inlet opening (11) ending in the trachea and an outlet opening (12) ending in the esophagus, and with an annular valve seat (2a) arranged in the range of the outlet opening (12) provided within the base body (2), the central circular passage opening (6) of said valve seat (2a) being opened and closed by a valve flap (3) operating as a non-return valve in the form of a one-way valve, a first external flange (1) arranged at the outlet side of the base body (2) and laterally projecting therefrom, said flange being adapted to fit to the interior wall of the esophagus, and a second external flange (5) arranged at the inlet side of the base body (2) and laterally projecting therefrom, said flange (5) being adapted to fit to the interior wall of the trachea, **characterized in that** the base body (2) consists of a hard and rigid material and the flanges (1, 5, 21, 31) as well as the valve flap (3) consist of soft and elastic materials.

2. Voice prosthesis (10, 20, 30) as claimed in claim 1, **characterized in that** the base body (2) and the flanges (1, 5, 21, 31) are produced as an all-around injection moulding unit.

3. Voice prosthesis (10, 20, 30) as claimed in claim 1 or 2, **characterized in that** the base body (2) consists of hard polyurethane and the flanges (1, 5, 21, 31) as well as the valve flap (3) consist of soft polyurethane, whereby the condition that the combination of materials has to enter a sufficiently stable connection in the course of the all-around injection moulding has to be satisfied.

4. Voice prosthesis (10, 20, 30) as claimed in claim 1 or 2, **characterized in that** base body (2) consists of polyethylene or of polypropylene and the flanges (1, 5, 21, 31) as well as the valve flap (3) consist of a thermoplastic elastomere.

5. Voice prosthesis (10, 20, 30) as claimed in one or in more than one of the claims 1 to 4, **characterized in that** the first flange (1, 21, 31) and the second flange (5) consist of respective different flexible materials.

6. Voice prosthesis (10, 20, 30) as claimed in one or in more than one of the claims 1 to 5, **characterized in that** the flanges (1, 5, 21, 31) are attached by injection moulding to the rigid base body (2).

7. Voice prosthesis (10, 20, 30) as claimed in one or in more than one of the claims 1 to 6, **characterized in that** the base body (2) is extended on its exit side by an axial portion (1a, 31a) of the laterally projecting first external flange (1, 31) and is closed on its inlet side by the laterally projecting second external flange (5).

8. Voice prosthesis (10, 20, 30) as claimed in one or in more than one of the claims 1 to 7, **characterized in that** the rigid base body (2) may be embodied with a variable wall thickness, in order to provide the base body (2), when small fistula sizes are concerned, with the possibly smallest outside diameter (B) under maintaining a required minimal interior diameter (A).

9. Voice prosthesis (10, 20) as claimed in one or in more than one of the claims 1 to 8, **characterized in that** the flanges (1, 5, 21) as well as the valve flap (3) are made of different flexible materials.

10. Voice prosthesis (10, 20) as claimed in one or in more than one of the claims 1 to 9, **characterized in that** the valve flap (3) is provided with a tongue-like end (7), which is passed through a slot (4) of the rigid base body (2) and is secured thereto by mechanical adhering or by a thermal deformation (7a) similar to a rivet.

11. Voice prosthesis (10, 20) as claimed in one or in more than one of the claims 1 to 9, **characterized in that** the valve flap (3), embodied with the tongue-like end (7), is fixed by injection moulding within the slot (4) of the rigid base body (2).

12. Voice prosthesis (10, 20) as claimed in one or in more than one of the claims 1 to 11, **characterized in that** the annular valve seat (2a) is manufactured as an integral part of the base body (2).

13. Voice prosthesis (20) as claimed in one or in more than one of the claims 1 to 12, **characterized in that** the base body (2) on its exit side is limited by a conical protective ring (22) having an umbrella-like flange (21) projecting to the outside at an acute angle, said flange being of a : particularly flexible material and protecting the valve flap (3) and the valve seat against ambient influences in each position of the voice prosthesis within the shunt.

14. Voice prosthesis (20) as claimed in one or in more than one of the claims 1 to 13, **characterized in that** the base body (2) is provided, in its interior wall within the range of the valve flap (3) above the securing slot (4), with a protective chamber (23) for receiving the valve flap (3).

15. Voice prosthesis (20) as claimed in one or in more than one of the claims 1 to 14, **characterized in that** the base body (2) is provided in its interior with an annular end support (24) for slide-in limitation of a preferably curved insertion rod (40).

16. Voice prosthesis (20) as claimed in one or in more than one of the claims 1 to 15, **characterized in that** the valve seat (2a) is provided with worked-in edged sealing grooves (25) in the sealing range of the valve flap (3).

17. Voice prosthesis (30) as claimed in one or in more than one of the claims 1 to 8, **characterized in that** the valve flap (3) with its valve flap end (7) are material homogeneously manufactured together with the upper exit side flange (31) by injection moulding and, when the voice prosthesis (30) is in the closed position, sealingly rests upon the upper seating face (34) of the base body (2).

18. Voice prosthesis (30) as claimed in claim 17, **characterized in that** the base body (2) is provided with a stepless interior space.

19. Voice prosthesis (30) as claimed in claims 17 or 18, **characterized in that** the base body (2) is provided on its outside surface with an annular notch (32') and (32"), respectively, for receiving an annular recess (31b) and (33), respectively, of the flanges (5, 31).

## Revendications

1. La prothèse vocale (10, 20, 30) destinée à être insérée dans le shunt situé entre la trachée et l'oesophage chez un patient laryngectomé, composée d'un corps tubulaire cylindrique (2) doté d'un orifice d'entrée (11) débouchant dans la trachée et d'un orifice de sortie (12) débouchant dans l'oesophage et d'un logement de valve circulaire (2a) disposé dans l'espace de l'orifice de sortie (12) au sein du corps de base, dont l'orifice de passage circulaire positionné de manière médiane (6) s'ouvre et se ferme à l'aide d'un clapet de valve (3) faisant fonction de valve-clapet anti-retour en forme de valve unidirectionnelle, d'une première bride extérieure en position écartée, côté sortie, sur le corps de base (2), pour assurer l'application sur la paroi intérieure de l'oesophage, et d'une deuxième bride extérieure (5) en position écartée, côté entrée, sur le corps de base (2), pour assurer l'application sur la paroi intérieure de la trachée, est **caractérisée en ce que** le corps de base (2) est fait dans un matériau dur et rigide et que les brides (1, 5, 21, 31) ainsi que le clapet de la valve (3) sont faits dans des matériaux souples et doux.

2. La prothèse vocale (10, 20, 30) suivant la revendication 1 est **caractérisée en ce que** le corps de base (2) et les brides (1, 5, 21, 31) sont fabriqués dans une unité de surmoulage plastique.

3. La prothèse vocale (10, 20, 30) suivant la revendication 1 est **caractérisée en ce que** le corps de base (2) est en polyuréthane dur et que les brides (1, 5, 21, 31) ainsi que le clapet de valve (3) sont en polyuréthane souple à condition toutefois de satisfaire à l'exigence de ce que les constituants de cette combinaison de matériaux deviennent suffisamment stables lors du surmoulage plastique.

4. La prothèse vocale (10, 20, 30) suivant les revendications 1 ou 2 est **caractérisée en ce que** le matériau utilisé pour réaliser le corps de base (2) est un polyéthylène ou un polypropylène et que le matériau utilisé pour les brides (1, 5, 21, 31) ainsi que pour le clapet des valve (3) est un élastomère thermoplastique.

5. La prothèse vocale (10, 20, 30) suivant une ou plusieurs des revendications 1 à 4 est **caractérisée en ce que** la première bride (1, 21, 31) et la deuxième bride (5) sont chacune composées de matériaux de souplesse différente.

6. La prothèse vocale (10, 20, 30) suivant une ou plusieurs des revendications 1 à 5 est **caractérisée en ce que** les brides (1, 5, 21, 31 ) sont fixées sur le corps de base rigide (2) par surmoulage.

7. La prothèse vocale (10, 20, 30) suivant une ou plusieurs des revendications 1 à 6 est **caractérisée en ce que** côté sortie, le corps de base (2) est prolongé par une pièce axiale (1a, 31 a) de la première bride extérieure (1,31) écartée latéralement et que, côté entrée, le corps de base est obstrué par la deuxième bride extérieure, également écartée latéralement.

8. La prothèse vocale (10, 20, 30) suivant une ou plusieurs des revendications 1 à 7 est **caractérisée en ce que** le corps de base rigide (2) se distingue par une épaisseur variable des parois pour permettre pour les petites fistules de pouvoir réaliser un corps de base (2) doté d'un diamètre extérieur (B) aussi petit que possible garantissant ainsi un diamètre intérieur (A) suffisamment réduit.

9. La prothèse vocale (10, 20,) suivant une ou plusieurs des revendications 1 à 8 est **caractérisée en ce que** les brides (1, 5, 21) ainsi que le clapet de valve (3) sont exécutés dans différents matériaux élastiques.

10. La prothèse vocale (10, 20,) suivant une ou plusieurs des revendications 1 à 9 est **caractérisée en ce que** la conception du clapet de valve (3) prévoit de donner à une extrémité (7) la forme d'une languette pour la faire passer par une fente (4) du corps de base rigide (2) afin de l'y fixer mécaniquement par collage ou au moyen de formage thermique par l'application d'une sorte de tête de rivet (7a).

11. La prothèse vocale (10, 20,) suivant une ou plusieurs des revendications 1 à 9 est **caractérisée en ce que** le clapet de la valve (3) avec son extrémité (7) en forme de languette est fixé par injection dans une fente (4) du corps de base (2) rigide.

12. La prothèse vocale (10, 20,) suivant une ou plusieurs des revendications 1 à 11 est **caractérisée en ce que** le logement circulaire de la valve (2a) est fabriqué de manière solidaire avec le corps de base (2), formant ainsi une seule pièce.

13. La prothèse vocale (20) suivant une ou plusieurs des revendications 1 à 12 est **caractérisée en ce que**, du côté sortie, le corps de base (2) est limité par une bague protectrice conique (22) pourvue d'une bride (21) qui, réalisée dans un matériau particulièrement souple, fait saillie dans angle aigu orienté vers l'extérieur à la manière d'un parapluie. Cette conception permet dans n'importe quelle position de la prothèse vocale de protéger dans le shunt le clapet de la valve (3) et son logement contre toute influence venant de l'extérieur.

14. La prothèse vocale (20) suivant une ou plusieurs des revendications 1 à 13 est **caractérisée en ce que** le corps de base (2) est muni sur sa paroi intérieure dans la zone du clapet de la valve (3) au-dessus de la fente de fixation (4) d'un espace protecteur (23) pour recevoir le clapet de valve (3).

15. La prothèse vocale (20) suivant une ou plusieurs des revendications 1 à 14 est **caractérisée en ce que** le corps de base (2) présente à l'intérieur une butée circulaire (24) destinée à limiter la profondeur d'introduction d'une barre d'introduction (40) de forme de préférence courbée.

16. La prothèse vocale (20) suivant une ou plusieurs des revendications 1 à 15 est **caractérisée en ce que** dans l'espace assurant l'étanchéité du clapet de la valve (3) le logement de la valve (2a) est pourvu de rainures (25).

17. La prothèse vocale (30) suivant une ou plusieurs des revendications 1 à 8 est **caractérisée en ce que** le clapet de la valve (3) et l'extrémité du clapet de la valve (7) sont solidairement réalisés dans le même matériau par surmoulage plastique avec la bride supérieure (31) située côté sortie et qu'ils reposent en position de fermeture de la prothèse vocale (30), sur la face frontale supérieure (34) du corps de base (2), afin d'en assurer l'étanchéité.

18. La prothèse vocale (30) suivant la revendication 17 est **caractérisée en ce que** le corps de base (2) présente un espace intérieur exempt de gradins.

19. La prothèse vocale (30) suivant la revendication 17 ou 18 est **caractérisée en ce que** le corps de base (2) est doté sur sa circonférence d'une rainure circulaire (32', 32") afin de pouvoir recevoir la gorge circulaire (31b, 33) des brides (5, 31).
